# EUROPEAN PATENT APPLICATION

(11) **EP 3 245 934 A1**
(43) Date of publication of application: **22.11.2017**
(21) Application number: 15877837.3
(22) Date of filing: 15.01.2015
(51) Int. Cl.: A61B 1/04

(54) **ENDOSCOPE AND IMAGING DEVICE**

(71) Applicant: OLYMPUS CORPORATION, Hachioji-shi Tokyo 192-8507 (JP)
(72) Inventor: FUJIMORI, Noriyuki, Tokyo 192-8507 (JP)
(74) Representative: Winter, Brandl, Fürniss, Hübner, Röss, Kaiser, Polte - Partnerschaft mbB
(86) International application number: PCT/JP2015/050972
(87) International publication number: WO 2016/113885

(57) **Abstract**

An endoscope 2 is an endoscope 2 in which an image pickup apparatus 1 is placed in a distal end portion 2a of an insertion portion, the image pickup apparatus 1 including an image pickup device 10 including a semiconductor substrate 11 in which an image pickup section 13 is formed on a first principal surface 10SA, the semiconductor substrate 11 including a bonding terminal 18 on a second principal surface 10SB, and a wiring layer 12 that is placed on the first principal surface 10SA of the semiconductor substrate 11 and includes a conductor layer 12A stacked via insulating layers 12B and 12C, and a cover glass 30 that is bonded in such a manner as to cover an entire side of the first principal surface of the image pickup device 10, the endoscope 2 including a protection section 40 configured to cover at least a side surface of the wiring layer 12 of the image pickup device 10, the protection section 40 including a resin 41 in which blocking particles 42 are dispersed, the blocking particles 42 having lower moisture permeability than moisture permeability of the resin 41.

## Description

### Technical Field

The present invention relates to an endoscope including an image pickup apparatus having a low dielectric constant material in a wiring layer, and the image pickup apparatus.

### Background Art

A chip-size package type image pickup apparatus including an image pickup device in which a light receiving section formed of a CMOS light receiving element or the like is formed on a principal surface (a light receiving surface) is small in diameter, and therefore is used in an endoscope. In order to achieve compatibility of the light receiving section formed of a fine pattern produced by a semiconductor technique, and a large bonding electrode to which a wiring board and the like are connected, a wiring layer formed of a conductor layer and an insulating layer is indispensable for the image pickup device. In recent years, in order to enhance performance of an image pickup apparatus, use of a material with a lower dielectric constant than silicon oxide, a so-called Low-k material as the insulating layer of the wiring layer has been considered.

However, a Low-k material is inferior to a conventional insulating layer material in moisture resistance/water resistance, that is, resistance to penetration of water (including water vapor and the like). A chip size package type image pickup apparatus having a Low-k material as an insulating layer has been likely to be insufficient in reliability, because the Low-k material is exposed on the outer circumferential portion. That is, there has been a concern that if water enters into the insulating layer formed from a Low-k material, problems may arise that a relative dielectric constant rises, parasitic capacitance increases and a signal delay occurs, whereby a malfunction occurs, and the insulating layer is detached.

Japanese Patent Application Laid-Open Publication No. 2011-166080 discloses an image pickup apparatus in which an image pickup device is housed in a shield case. A sealing resin is filled in a gap between the image pickup device and the shield case.

However, the above described publication neither discloses nor suggests anything about an influence by water that penetrates via the sealing resin. It is inferred that this is because a Low-k material is not used as the insulating layer of the image pickup device.

### Citation List

### Patent Literature

Patent Literature 1: Japanese Patent Application Laid-Open Publication No. 2011-166080

### Disclosure of Invention

### Technical Problem

An embodiment of the present invention has an object to provide an endoscope that is excellent in moisture resistance and has high reliability, and an image pickup apparatus that is excellent in humidity resistance and has high reliability.

### Solution to Problem

An endoscope of an embodiment of the present invention is an endoscope in which an image pickup apparatus is placed in a distal end portion of an insertion portion, the image pickup apparatus including an image pickup device including a semiconductor substrate in which a light receiving section is formed on a first principal surface, the semiconductor substrate including a bonding terminal electrically connected to the light receiving section on a second principal surface, and a wiring layer that is placed on the first principal surface of the semiconductor substrate and includes a conductor layer stacked via an insulating layer, and a cover glass that is bonded in such a manner as to cover an entire side of the first principal surface of the image pickup device, the endoscope including a protection section configured to cover at least a side surface of the wiring layer of the image pickup device, the protection section including a resin in which blocking particles are dispersed, the blocking particles having lower moisture permeability than moisture permeability of the resin.

An image pickup apparatus of another embodiment is an image pickup apparatus including an image pickup device including a semiconductor substrate in which a light receiving section is formed on a first principal surface, the semiconductor substrate including a bonding terminal electrically connected to the light receiving section on a second principal surface, and a wiring layer that is placed on the first principal surface of the semiconductor substrate and includes a conductor layer stacked via an insulating layer, and a cover glass that is bonded in such a manner as to cover an entire side of the first principal surface of the image pickup device, the image pickup apparatus including a protection section configured to cover at least a side surface of the wiring layer of the image pickup device, the protection section including a resin in which blocking particles are dispersed, the blocking particles having lower moisture permeability than moisture permeability of the resin.

### Advantageous Effect of Invention

According to the present invention, the endoscope that is excellent in moisture resistance and has high reliability, and the image pickup apparatus that is excellent in humidity resistance and has high reliability can be provided.

### Brief Description of the Drawings

Fig. 1 is a view for explaining an endoscope system including an endoscope of a first embodiment;
Fig. 2 is a perspective view of an image pickup apparatus of the endoscope of the first embodiment;
Fig. 3 is a top view of the image pickup apparatus of the endoscope of the first embodiment;
Fig. 4 is a sectional view taken along line IV-IV in Fig. 3, of the image pickup apparatus of the endoscope of the first embodiment;
Fig. 5 is a sectional view of an image pickup unit of the endoscope of the first embodiment;
Fig. 6 is a sectional view of a protection section of the endoscope of the first embodiment;
Fig. 7 is a schematic sectional view of the protection section of the endoscope of the first embodiment;
Fig. 8 is a sectional view of an image pickup unit of an endoscope of modification 1 of the first embodiment;
Fig. 9 is a schematic sectional view of a protection section of a modification of an endoscope of modification 2 of the first embodiment;
Fig. 10 is a sectional view of an image pickup unit of an endoscope of modification 3 of the first embodiment;
Fig. 11 is a schematic sectional view of a protection section of an endoscope of a second embodiment;
Fig. 12 is a schematic sectional view of a protection section of an endoscope of modification 1 of the second embodiment; and
Fig. 13 is a sectional view of an image pickup unit of an endoscope of modification 2 of the second embodiment.

### Best Mode for Carrying Out the Invention

### <First Embodiment>

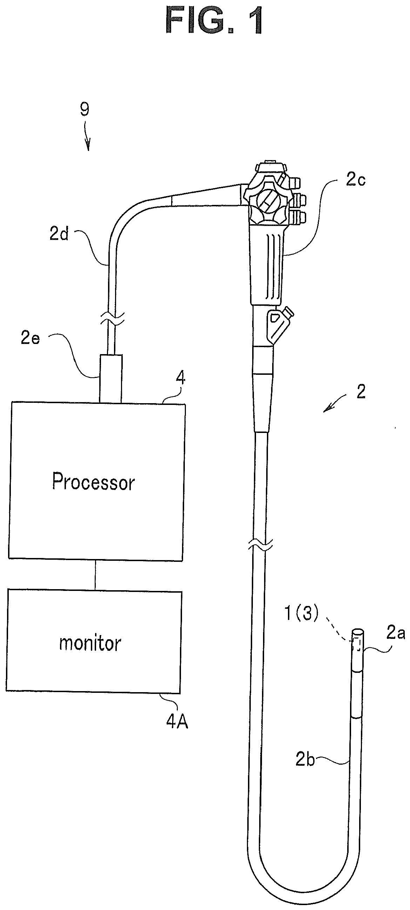

As illustrated in Fig. 1, an endoscope 2 of the present embodiment configures an endoscope system 9, with a processor 4. The endoscope 2 has a distal end portion 2a in which an image pickup unit 3 including an image pickup apparatus 1 is placed, an elongated insertion portion 2b provided extensively from the distal end portion 2a, an operation portion 2c placed at a proximal end portion side of the insertion portion 2b, a universal cord 2d that is provided extensively from the operation portion 2c, and a connector 2e placed at a proximal end portion side of the universal cord 2d.

The connector 2e is attachably and detachably connected to the processor 4. The processor 4 performs signal processing of an image pickup signal of the image pickup apparatus 1 and outputs an image signal to a monitor 4A.

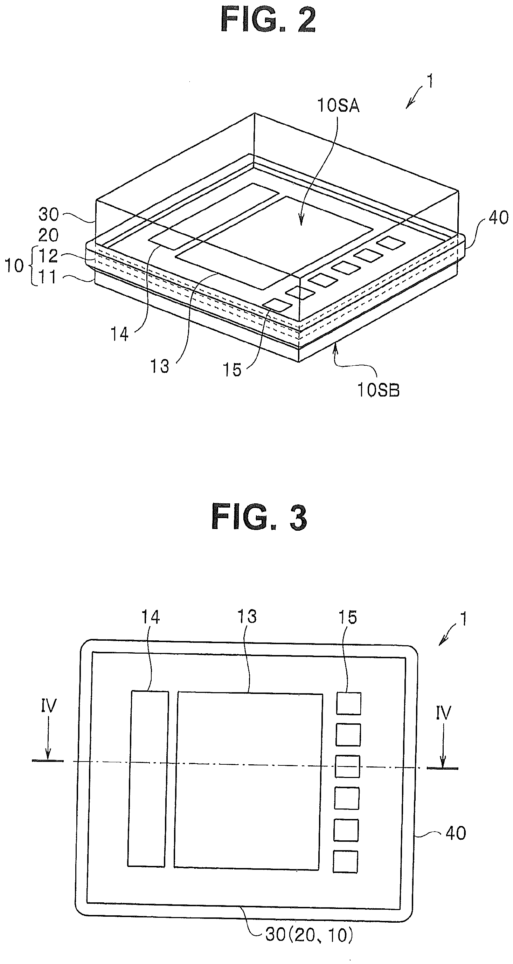

As illustrated in Fig. 2 to Fig. 4, the image pickup apparatus 1 includes an image pickup device 10, a cover glass 30 that is a transparent member, and an adhesive layer 20 that bonds the image pickup device 10 and the cover glass 30. The image pickup device 10 includes a semiconductor substrate 11 on which an image pickup section (a light receiving section/ a pixel section) 13 is formed and a wiring layer 12. As will be described later, a side surface of the wiring layer 12 is surrounded by a protection section 40 of a specific configuration, and therefore the image pickup apparatus 1 is excellent in moisture resistance.

Note that the drawings are schematic, and attention should be paid to that relationships among thicknesses and widths of respective portions, ratios of the thicknesses of the respective portions, sizes and content rates of particles, and the like differ from the relationships, the ratios, sizes, content rates and the like in reality, and parts in which the relationships and the ratios of mutual dimensions differ may be included among the drawings. Further, shapes of the particles in sectional views are not sectional shapes but side shapes, and the sizes of the particles significantly differ from actual sizes for convenience of illustration.

On a first principal surface 10SA of the image pickup device 10, the image pickup section 13, a circuit section 14, and a plurality of electrode pads 15 are formed. On a second principal surface 10SB of the image pickup device 10, a plurality of bonding terminals 18 that are connected to the respective electrode pads 15 via respective through wirings 17 are formed.

The image pickup section 13 formed of a CMOS image pickup device and the like is formed on a principal surface of the semiconductor substrate 11 formed from silicon or the like by a known semiconductor production technique.

The circuit section 14 includes a semiconductor circuit that processes a signal of the image pickup section 13. The semiconductor circuit is formed on the principal surface of the semiconductor substrate 11 formed from silicon or the like by the known semiconductor production technique similarly to the image pickup section 13.

The image pickup device 10 formed of the semiconductor substrate 11 in which the wiring layer 12 is placed on the first principal surface 10SA is a chip of a wafer level chip size package type, and the image pickup device 10 and the cover glass 30 have a same size in plan view. That is, the image pickup apparatuses 1 are manufactured by cutting and singulating a bonded wafer in which an image pickup device wafer where a plurality of image pickup devices 10 are formed and a glass wafer are bonded. The image pickup device 10 can be mass-produced in a collective manner, and therefore is excellent in productivity.

The image pickup apparatus 1 does not have to be necessarily formed of a wafer level chip size package type image pickup device chip as a matter of course, but may be produced by bonding the image pickup device 10 that is already singulated and the cover glass 30.

At least one insulating layer 12C of the wiring layer 12 is formed from a low dielectric constant material (a Low-k material). The electrode pad 15 is connected to the image pickup section 13 and the circuit section 14 via the wiring layer 12.

Note that the conductive materials of the plurality of conductor layers 12A may be formed of different materials. Further, insulating materials of a plurality of insulating layers 12B may be formed of different materials. At least the one insulating layer 12C is formed from a low dielectric constant material (a Low-k material).

A low dielectric constant material refers to a material which is lower in relative dielectric constant k than a silicon oxide (k=4.0), and is preferably a material with the relative dielectric constant k of 3.0 or less. A lower limit value of the relative dielectric constant k of the low dielectric constant material is 2.0 or more, and is preferably 1.5 or more, due to technical limitations.

In the image pickup apparatus 1, the low dielectric constant material of the insulating layer 12C is a porous carbon-doped silicon oxide film (SiOC). A porous SiOC is structurally formed as a porous substance having voids, and is capable of having the relative dielectric constant k of 2.7.

As the material of the insulating layer 12C, a fluorine-doped silicon oxide film (SiOF/FSG), a hydrogen-containing polysiloxane (HSQ) material, a methyl-containing polysiloxane (MSQ) material, organic (a polyimide, a parylene, a fluorine resin) materials and the like are usable, besides SiOC.

The cover glass 30 which is a transparent member that is bonded to the first principal surface 10SA of the image pickup device 10 via the adhesive layer 20 has a same size in plan view as the image pickup device 10. The transparent member may be formed from a resin or the like as long as the material has a high transmissivity in a wavelength region of light received by the image pickup section 13. The cover glass 30 having a sufficient thickness blocks penetration of water to the image pickup section 13, the circuit section 14 and the like from an upper part.

The adhesive layer 20 is formed from an epoxy resin, a silicone resin or the like of an ultraviolet curing type or a thermosetting type that is more excellent in moisture resistance than the low dielectric constant material of the insulating layer 12C. Note that when a micro lens array is placed directly on the image pickup section 13, the adhesive layer 20 does not have to be placed.

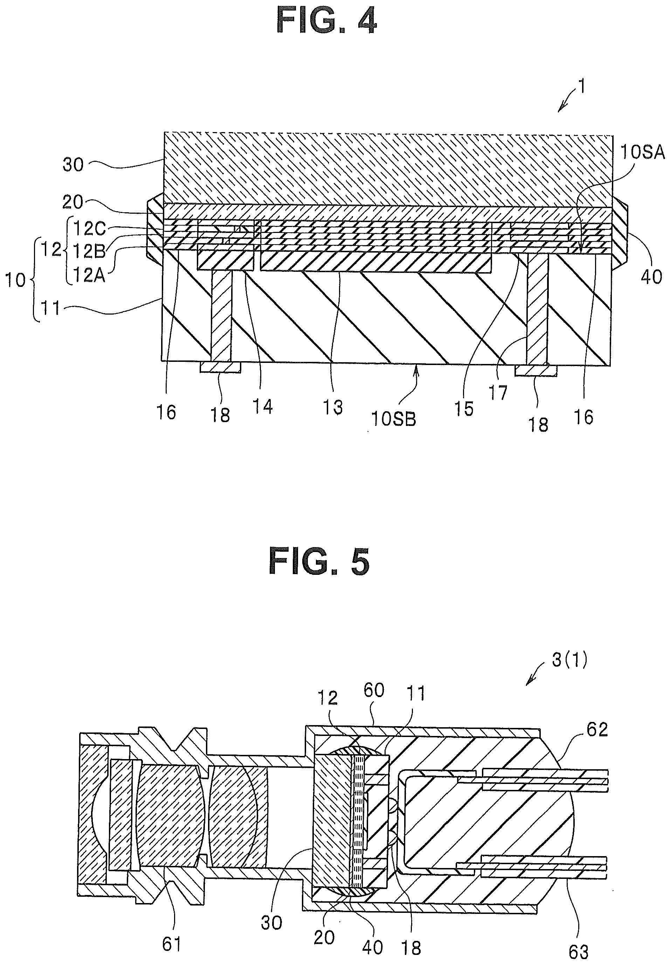

As illustrated in Fig. 5, the image pickup apparatus 1 is housed inside a shield case 60 to which an objective lens optical system 61 is fixed, and is placed in the endoscope 2 as an image pickup unit 3. The bonding terminal 18 of the image pickup apparatus 1 is electrically connected to a signal cable 63 via a wiring board 64. The signal cable 63 is connected to the processor 4 that performs control and the like of the image pickup apparatus and processes the image pickup signals simultaneously.

A gap between a side surface of the image pickup device 10 and an inner wall of the shield case 60 is sealed by a sealing resin 62. As for the sealing resin 62, a resin that is excellent in moisture resistance is selected, such as an ultraviolet curing type or a thermosetting type epoxy resin or silicone resin. The shield case 60 is formed from a metal such as a stainless steel, has a light blocking function and an electromagnetic noise resistance improving function, and has moisture resistance preventing penetration of water. Note that as will be described later, in order to reduce the diameter of the endoscope 2, the image pickup unit may be an image pickup unit that does not include the shield case 60.

As in the above explanation, a side surface of the wiring layer 12 is protected with the sealing resin 62 and the shield case 60. However, in order to prevent entry of water into the wiring layer 12 more reliably, the protection section 40 is placed in the image pickup apparatus 1.

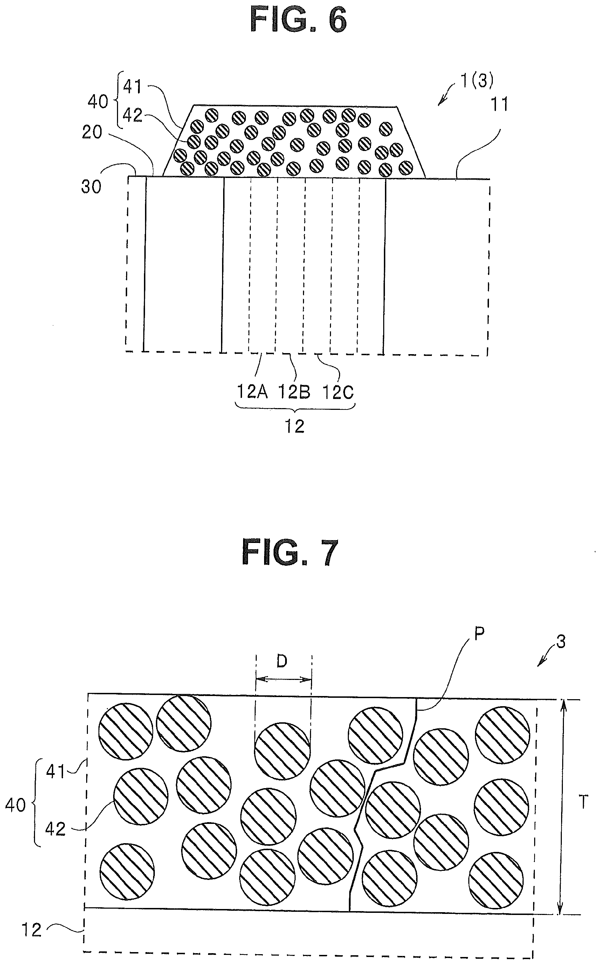

As illustrated in Fig. 6, in the protection section 40, blocking particles 42 having lower moisture permeability than a resin 41 are dispersed in the resin 41. The moisture permeability was evaluated by JIS Z 0208 (cup method) by producing test films of a predetermined thickness. For example, from comparison of moisture permeability of a test film formed from only the resin 41, and moisture permeability of a test film formed from the resin 41 in which the particles are dispersed, it can be confirmed that the moisture permeability of the particles is lower than the moisture permeability of the resin 41.

For example, when an epoxy resin or a silicone resin is used as the resin, use of the blocking particles 42 formed from silica or alumina is preferable.

Further, when a diameter D of the blocking particle 42 is from 1/100 to 1/5 inclusive of a thickness T of the protection section 40, moisture permeability is improved significantly (refer to Fig. 7). The thickness T of the protection section 40 is a value measured from a photograph obtained by photographing a section with an electron microscope, and is an arithmetic means value when irregularities are present. The diameter D of the particle is an arithmetic means value measured from a photograph obtained by photographing the particles before dispersion with an electron microscope. The blocking particle 42 (microscopic powder, a filler) may be in an elliptical shape, a rectangular shape, a rod-shape, a fibriform, an amorphous state or the like instead of a spherical shape. Further, the diameters D of the particles may have a predetermined distribution. Furthermore, although the protection section 40 may include particles of different particle sizes, 90% by weight or more of the particles are preferably within the aforementioned range, as a ratio in the blocking particles 42.

It is estimated that the protection section 40 in which the blocking particles 42 having lower moisture permeability than the resin 41 are dispersed can effectively prevent entry of water, because an entry path P of water becomes long due to the blocking particles 42, as illustrated in Fig. 7. When the diameter D of the blocking particle 42 is a lower limit of the aforementioned range or more, a predetermined moisture permeability improvement effect is confirmed, whereas when the diameter D is an upper limit of the aforementioned range or less, dispersibility of the blocking particles 42 in the resin 41 is favorable, and filling and the like are facilitated.

Note that in order to obtain the above described effect more effectively, a content rate of the blocking particles 42 is preferably 30% by weight or more, and is especially preferably 75% by weight or more. However, when the particle content rate is too high, bonding strength to the substrate is reduced, so that the particle content rate is preferably 95% by weight or less, and is especially preferably 90% by weight or less. Note that the particle content rate is a preparation value of a dispersion solution to be coated.

In the wiring layer 12 of the image pickup apparatus 1, penetration of water from the side surface is blocked by the protection section 40, the sealing resin 62 and the shield case 60. Consequently, the image pickup apparatus 1 includes high reliability even though the image pickup apparatus 1 is a chip size package type semiconductor device with a small size in plan view, and has a configuration in which the low dielectric constant material (a Low-k material) is exposed on the side surface of the wiring layer 12.

Even when the image pickup apparatus 1 was left in a high-temperature and high-humidity environment at 85°C with humidity of 85% for 1000 hours, for example, characteristics of the image pickup apparatus 1 were not deteriorated. Further, performance of the endoscope 2 in which the image pickup apparatus 1 is placed was not impaired even after predetermined sterilization treatment and the like were applied.

### <Modifications of First Embodiment>

Endoscopes of modifications of the first embodiment that will be described next are analogous to the endoscope 2 of the first embodiment and have the effect of the endoscope 2, so that components having same functions are assigned with same reference numerals and explanation will be omitted.

In the endoscope 2, the protection section 40 covers the side surface of the wiring layer 12. However, the protection section can cover the side surface of at least the wiring layer 12, in other words, the protection section 40 may cover not only the side surface of the wiring layer 12, but also side surfaces of the cover glass 30, the semiconductor substrate 11 and the adhesive layer 20 and the like.

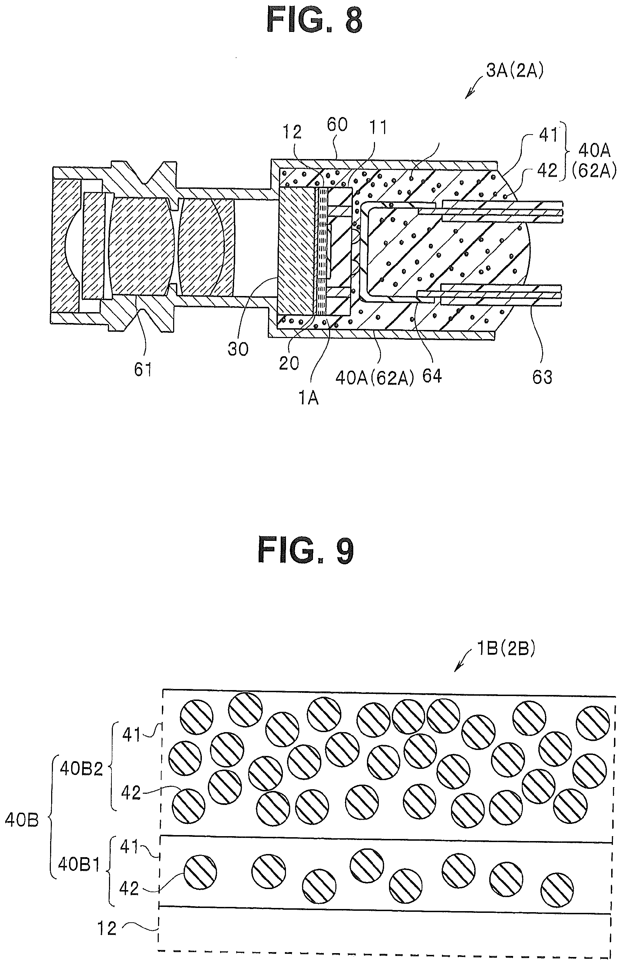

In an image pickup unit 3A of an endoscope 2A of modification 1 of the first embodiment illustrated in Fig. 8, for example, not only the side surface of the wiring layer 12 of an image pickup apparatus 1A but also an interior of the shield case 60 is sealed by a protection section 40A that is formed from the resin 41 including the blocking particles 42 similarly to the protection section 40. In other words, a sealing resin 62A has a configuration in which the blocking particles 42 having lower moisture permeability than the permeability of the resin are dispersed in the resin 41.

A protection section 40B of an image pickup apparatus 1B of an endoscope 2B of modification 2 of the first embodiment that will be described next covers the side surface of the wiring layer 12 as in the image pickup apparatus 1 of the first embodiment.

However, as illustrated in Fig. 9, in the protection section 40B, a content rate of the blocking particles 42 changes in a thickness direction, and a particle content rate of a first protection section 40B1 at an inner side that contacts the wiring layer 12 is smaller than a particle content rate of a second protection section 40B2 at an outer side.

The protection section 40B is formed of the first protection section 40B1 and the second protection section 40B2 having different particle content rates, so that bonding strength to the wiring layer 12 can be made sufficiently high, and the content rate of the blocking particles 42 of the protection section 40B can be made large.

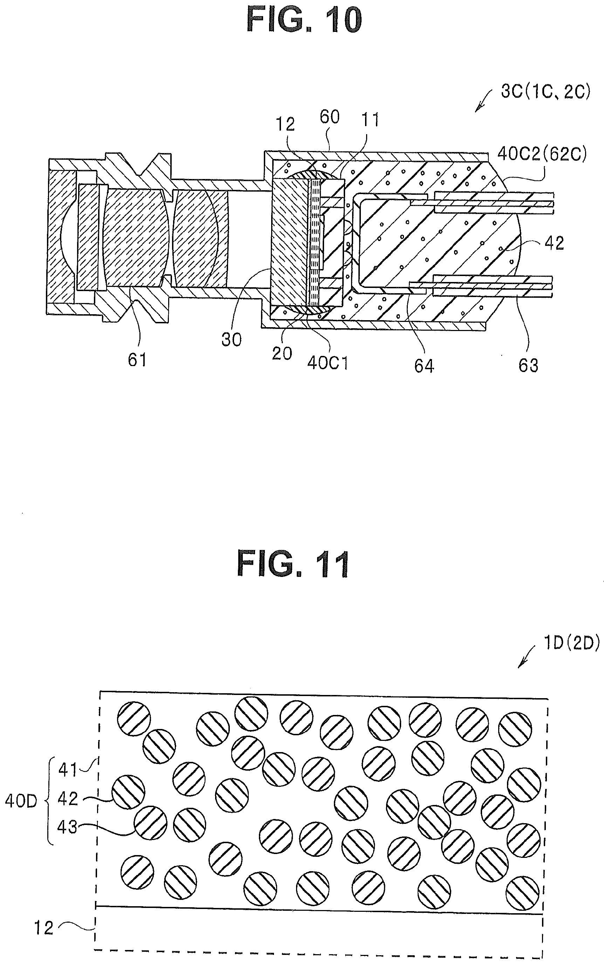

Furthermore, in an image pickup unit 3C including an image pickup apparatus 1C of an endoscope 2C of modification 3 of the first embodiment illustrated in Fig. 10, the side surface of the wiring layer 12 is covered with a first protection section 40C1, and the interior of the shield case 60 is sealed by a sealing resin 62C that is a second protection section 40C2 having a larger particle content rate than the first protection section 40C1.

Note that when the content rate of the blocking particles 42 changes as in modifications 2 and 3, a content rate in a highest region is preferably from 30% by weight to 95% by weight inclusive, and is especially preferably 50% by weight to 90% by weight inclusive. The content rate in the lowest region may be 0%. However, a content rate of the entire protection section is preferably 30% by weight or more.

Note that the protection section in which the content rate of the blocking particles 42 changes in the thickness direction may be of a multilayer structure with three layers or more, or may be a composition gradient film.

Since the image pickup unit 3C is formed of the first protection section 40C1 and the second protection section 40C2 having different particle content rates, the bonding strength to the wiring layer 12 can be made sufficiently high, and the content rate of the blocking particles 42 in the protection section can be made large.

### <Second Embodiment>

Next, an endoscope 2D of a second embodiment will be described. The endoscope 2D is analogous to the endoscope 2 of the first embodiment, so that same components are assigned with same reference signs, and explanation will be omitted.

The endoscope 2D differs from the endoscope 2 of the first embodiment in a configuration of a protection section 40D.

As illustrated in Fig. 11, the protection section 40D of the image pickup apparatus 1D of the endoscope 2D includes moisture absorbing particles 43 having a higher moisture absorbing rate than the moisture absorbing rate of the resin 41, in addition to the blocking particles 42.

The moisture absorption rate was evaluated by JIS 7209. For example, when an epoxy resin or a silicone resin is used as the resin 41, the moisture absorbing particles 43 formed from carbon black, carbon nanotubes, bentonite, or zeolite is used.

The moisture absorbing particles 43 trap water that enters the resin 41. Consequently, the protection section 40D including the moisture absorbing particles 43 in addition to the blocking particles 42 has a higher entry prevention effect for water to the wiring layer 12 than the protection section 40. Therefore, the endoscope 2D has the effect of the endoscope 2, and has higher reliability.

That is, the distal end portion 2a of the endoscope 2D is exposed to a high-humidity environment only at a time of use or the like. For example, a medical endoscope would have a possibility of water entering while the medical endoscope is inserted into a body and during sterilizing treatment after use. The water trapped by the moisture absorbing particles 43 are released to outside when the endoscope is not in use, in particular, at a drying treatment time.

A content rate of the moisture absorbing particles 43 is preferably 5% by weight or more with which a moisture absorption improvement effect is remarkable, and an upper limit is 65% by weight. Further, a total of the content rate of the blocking particles 42 and the content rate of the moisture absorbing particles 43 is preferably from 30% by weight to 95% by weight inclusive.

For example, carbon black having a light blocking property is used as the moisture absorbing particles 43, and thereby incidence of external light onto the image pickup device can be prevented.

### <Modifications of Second Embodiment>

In an endoscope 2E of modification 1 of a second embodiment, a protection section 40E of an image pickup apparatus 1E is of a three-layer structure in which a first protection section 40E1 including the blocking particles 42, a second protection section 40E2 including the moisture absorbing particles 43 and a third protection section 40E3 including the blocking particles 42 are sequentially stacked in layer.

The particle content rate of the first protection section 40E1 that contacts the wiring layer 12 is smaller than the particle content rate in the third protection section 40E3 at an outer side. Consequently, the protection section 40E has high bonding strength. Furthermore, when water that penetrates into the third protection section 40E3 reaches the protection section 40E2, the water is trapped by the protection section 40E2. When some water is not fully trapped and passes through the protection section 40E2, the water is blocked by the protection section 40E1.

The endoscope 2E has the effect of the endoscope 2D and the like, and has higher reliability.

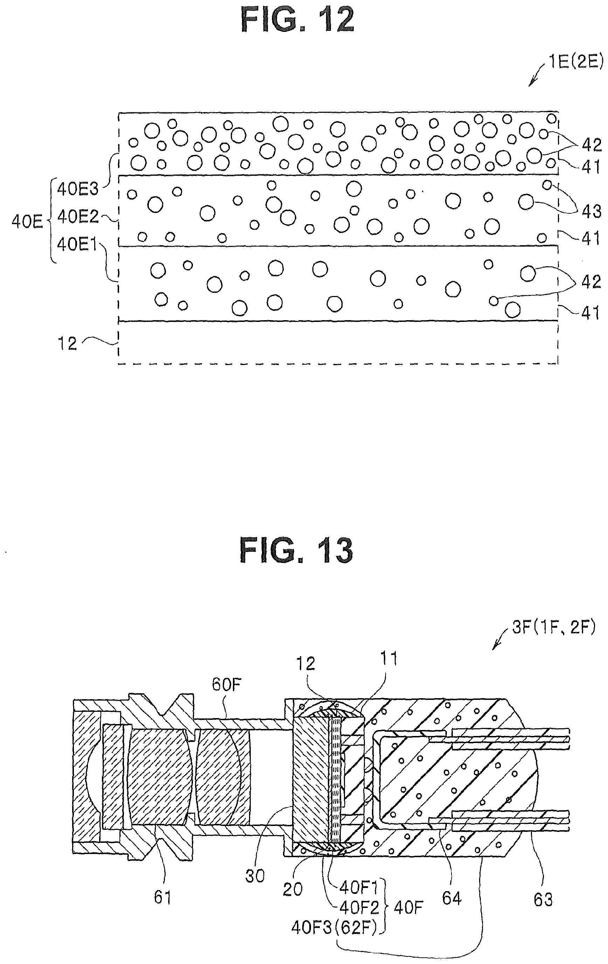

Next, in an endoscope 2F of modification 2 of the second embodiment illustrated in Fig. 13, in a protection section 40F of an image pickup apparatus 1F of an image pickup unit 3F, the side surface of the wiring layer 12 is covered with a first protection section 40F1, and further, the first protection section 40F1 is covered with a second protection section 40F2, and a third protection section 40F3 further seals a rear end portion of the image pickup apparatus as a sealing resin 62F.

Note that the image pickup unit 3F does not include a shield case, and the objective lens optical system 61 is fixed to a frame member 60F that is bonded to the cover glass 30.

The endoscope 2F has the effect of the endoscope 2E, and has higher reliability. Further, the endoscope 2F is reduced in diameter more easily than the endoscope including a shield case.

### <Image Pickup Apparatus>

Note that in the above explanation, the endoscopes in which the side surfaces of the wiring layers 12 of the image pickup apparatuses are covered with the protection sections 40 and 40A to 40F of specific configurations are described. However, it is needless to say that the image pickup apparatuses 1 and 1 to 1F can be used, as various image pickup apparatuses for use in environments requiring high moisture barrier characteristics.

For example, an image pickup apparatus as follows is excellent in moisture resistance, and has high reliability.

An image pickup apparatus including an image pickup device having a semiconductor substrate having a light receiving section formed on a first principal surface, and having a bonding terminal electrically connected to the light receiving section, on a second principal surface, and a wiring layer that is placed on the first principal surface of the semiconductor substrate, and has a conductor layer stacked via an insulating layer, and a cover glass that is bonded in such a manner as to cover a whole of the first principal surface side, of the image pickup device, the image pickup apparatus having a protection section configured to cover at least a side surface of the wiring layer of the image pickup device, and have blocking particles having lower moisture permeability than moisture permeability of a resin dispersed in the resin.

As above, the present invention is not limited to the aforementioned embodiments and the like, and various modifications, alterations and the like can be made within the range without departing from the gist of the present invention.

### Reference Signs List

1, 1A to 1F ... Image pickup apparatus
2, 2A to 2F ... Endoscope
3 ... Image pickup unit
9 ... Endoscope system
10 ... Image pickup device
11 ... Semiconductor substrate
12 ... Wiring layer
13 ... Image pickup section
14 ... Circuit section
15 ... Electrode pad
17 ... Through wiring
18 ... Bonding terminal
20 ... Adhesive layer
30 ... Cover glass
40 ... Protection section
41 ... Resin
42 ... Blocking particle
43 ... Moisture absorbing particle
50 ... Sealing resin
60 ... Shield case
62 ... Sealing resin

## Claims

1. An endoscope in which an image pickup apparatus is placed in a distal end portion of an insertion portion, the image pickup apparatus comprising:
an image pickup device including a semiconductor substrate in which a light receiving section is formed on a first principal surface, the semiconductor substrate including a bonding terminal electrically connected to the light receiving section on a second principal surface, and a wiring layer that is placed on the first principal surface of the semiconductor substrate and includes a conductor layer stacked via an insulating layer, and
a cover glass that is bonded in such a manner as to cover an entire side of the first principal surface of the image pickup device,
the endoscope comprising:
a protection section configured to cover at least a side surface of the wiring layer of the image pickup device, the protection section including a resin in which blocking particles are dispersed, the blocking particles having lower moisture permeability than moisture permeability of the resin.

2. The endoscope according to claim 1,
wherein the blocking particles are formed from silica or alumina.

3. The endoscope according to claim 1 or claim 2,
wherein diameters of the blocking particles are from 1/100 to 1/5 inclusive of a thickness of the protection section.

4. The endoscope according to any one of claim 1 to claim 3,
wherein a content rate of the blocking particles is from 30% by weight to 95% by weight inclusive.

5. The endoscope according to any one of claim 1 to claim 4,
wherein a content rate of the blocking particles changes in a thickness direction of the protection section, and
a particle content rate in a region at an outer side is larger than a particle content rate in a region at an inner side.

6. The endoscope according to any one of claim 1 to claim 5,
wherein the protection section includes moisture absorbing particles having a higher moisture absorption rate than a moisture absorption rate of the resin.

7. The endoscope according to claim 6,
wherein the moisture absorbing particles are formed from carbon black, carbon nanotubes, bentonite, or zeolite.

8. The endoscope according to claim 6 or claim 7,
wherein the moisture absorbing particles have light blocking properties.

9. The endoscope according to any one of claim 6 to claim 8,
wherein a content rate of the moisture absorbing particles is 5% by weight to 65% by weight inclusive, and
a total of a content rate of the blocking particles and the content rate of the moisture absorbing particles is 30% by weight to 95% by weight inclusive.

10. The endoscope according to any one of claim 6 to claim 9,
wherein a content rate of the blocking particles and a content rate of the moisture absorbing particles change in a thickness direction of the protection section.

11. The endoscope according to claim 10,
wherein the protection section is of a multilayer structure in which a first protection section including the blocking particles, a second protection section including the moisture absorbing particles, and a third protection section including the blocking particles and having a particle content rate larger than a particle content rate of the first protection section, are sequentially stacked in layers.

12. An image pickup apparatus comprising:
an image pickup device including a semiconductor substrate in which a light receiving section is formed on a first principal surface, the semiconductor substrate including a bonding terminal electrically connected to the light receiving section on a second principal surface, and a wiring layer that is placed on the first principal surface of the semiconductor substrate and includes a conductor layer stacked via an insulating layer, and
a cover glass that is bonded in such a manner as to cover an entire side of the first principal surface of the image pickup device,
the image pickup apparatus comprising a protection section configured to cover at least a side surface of the wiring layer of the image pickup device, the protection section including a resin in which blocking particles are dispersed, the blocking particles having lower moisture permeability than moisture permeability of the resin.
